# EUROPEAN PATENT APPLICATION

(11) **EP 4 548 903 A1**
(43) Date of publication of application: **07.05.2025**
(21) Application number: 23206597.9
(22) Date of filing: 30.10.2023
(51) Int. Cl.: A61K 8/02, A61K 8/26, A61Q 11/00

(54) **DENTIFRICE COMPOSITION COMPRISING FINE GROUND ALUMINA**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

The present invention relates to dentifrice compositions comprising fine ground alumina with a D90 particle size of less than 5 µm as an opacifier in an orally acceptable carrier.

## Description

### FIELD OF THE INVENTION

The present invention relates to dentifrice compositions comprising a fine ground alumina wherein the alumina has a D90 particle size of less than 5 µm and an orally acceptable carrier. The dentifrice compositions are highly opacified. The compositions may be free of titanium dioxide.

### BACKGROUND OF THE INVENTION

Oral care compositions, in particular dentifrices, are used to clean teeth on a daily basis. The most common form of dentifrice are pastes.

The pastes are made from a variety of different ingredients and are most often treated with a dye or pigment to give them an attractive appearance to consumers.

Titanium dioxide is the most widely used white pigment in toothpastes because of its brightness and very high refractive index (~2.6), in which it is surpassed only by a very few other materials. At low levels it produces a brilliant white colour which is much prized in many consumer products.

Despite its long term use in many applications, including food pigmentation, doubts and arguments regarding its potential toxicity to humans have lead to the use titanium dioxide being banned in food applications in the EU since February 2022. At least two US states are also currently considering banning the material on foods.

While toothpastes are not designed for consumption, they are used in the mouth. It would be desirable to find a replacement whitening additive free of the potential toxicity provided by titanium dioxide.

### SUMMARY OF THE INVENTION

In the broadest aspect of the present invention, the invention comprises a dentifrice composition comprising fine ground alumina with a D90 particle size of less than 5 µm and an orally acceptable carrier.

In a further embodiment the dentifrice composition the fine ground alumina has a D90 particle size of of less than 3 µm.

In a further embodiment the fine ground alumina has a bulk density of between 800 g and 1200 g per 1000 cm³.

In a further embodiment the the fina ground alumina comprises between 0.05 % and 15 % by weight.

In a further embodiment the fine ground alumina comprises between 0.1 % and 2 % by weight.

In a further embodiment the the fine ground alumina comprises between 0.2 % and 0.8 % by weight.

In a further embodiment the the dentifrice composition is either aqueous or non-aqueous.

In a further embodiment the the dentifrice composition comprises an abrasive.

In a further embodiment the the dentifrice composition comprises a water soluble condensed phosphate.

In a further embodiment the the dentifrice comprises at least one surfactant.

In a further embodiment the the dentifrice composition further comprises two or more surfactants.

In a further embodiment the surfactant comprises between 0.1 and 5% by weight of surfactant.

In a further embodiment the composition further comprises a desensitizing agent.

In a further embodiment the composition further further comprises a desensitising agent, an anti-bacterial agent, an anti-plaque agent, an anti-calculus agent, an oral malodour agent, an anti-inflammatory agent, an anti-oxidant, an anti-fungal agent, a wound healing agent or a mixture of at least two thereof
In a further embodiment the composition further comprises a source of fluoride ions.

In a further embodiment the composition is free of titanium dioxide.

### DETAILED DESCRIPTION OF THE INVENTION

The present invention is described in more detail below.

The following definitions should be considered in reviewing the description of the invention as set forth below.

As used herein, the word "comprising" encompasses "consisting of" and "consisting essentially of".

As used herein, the word "include", and its variants, is intended to be non-limiting such that recitation of items in a list is not to the exclusion of other items that may also be useful in the materials, compositions and methods of the invention.

All percentages used herein are by weight of the total dentifrice composition, unless otherwise specified.

As used throughout, ranges are used as a shorthand for describing each and every value that is within the range. Any value within the range can be selected as the terminus of the range.

Herein, "effective amount" means an amount of a given agent or material sufficient to provide a positive benefit, usually an oral health benefit.

As used herein, the word "about", when applied to a value for a parameter of a composition indicates that the calculation or measurement of the value allows some slight imprecision without having a substantial effect on the chemical or physical attributes of the composition.

The are many ways to define a particle size distribution. The cumulative distribution is particularly common and the most important way of defining this is by percentiles. These indicate in each case the size x below which a certain quantity of the sample lies.

Percentiles are denoted by the letter D followed by the % value. So for the purposes of the present invention the D90 value indicates that 90% of the particles of the alumina used are smaller than the size indicated.

A dentifrice composition is a product that is intended for application to the oral cavity for a period of time sufficient to contact all of the tooth surfaces and / or oral tissues for purposes of oral activity. As used herein, a "dentifrice composition" may mean a paste or gel formulation unless otherwise specified.

Aluminium oxide (or aluminium(III) oxide) is a chemical compound of aluminium and oxygen with the chemical formula Al₂O₃. It is the most commonly occurring of several aluminium oxides, and specifically identified as aluminium oxide. It is commonly called alumina and may also be called aloxide, aloxite, or alundum in various forms and applications.

The applicants have surprisingly found that finely ground alumina particles have a strong opacifying effect.

In particular, alumina with a D90 particle size of less than 5 µm has been found to have an excellent opacifying (whitening effect) properties in dentiftices, in particular in toothpastes.

Preferably the alumina may have a D90 particle size of less than 3 µm.

Preferably the alumina has a bulk density of between 800 g and 1200 g per 1000 cm³
The alumina may be used at smaller particle sizes if desired. A particularly preferred D90 particle size is around 2 µm.

Alumina has been known for use as an abrasive in dentifrices for decades. And is therefore known to be a safe ingredient.

Alumin added as an abrasive is typically used with paricle sizes greater than 10 µm. However, in the finely ground form of the present invention the alumina has been found to have no significant abrasive characteristics when added to a dentifrice composition.

The fine ground alumina of the present invention may be added in any amount desired to dentifrice compositions.

Preferablly the fine ground alumina will be added to dentifrice compositions between 0.05 % and 15 % by weight to provide optimal whitening (opacifying) performance.

In further embodiments, the fine ground alumina will be added to dentifrice compositions between 0.1 % and 2 % by weight and between 0.2 % and 0.8 % by weight.

The addition of the fine ground alumina to a dentifrice composition provides a strong opacifying (whitening appearance) effect to the dentifrice composition.

For a strong white appearance between 1.0 % to 2.0 % by weight, preferably between 1.2 % and 1.8% by weight of the fine ground alumina may be added to the dentifrice composition.

A possible commercial source of alumina of the invention is Ultimate P1800 ^{®} alumina from Almatis.

The dentifrice compositions may then be marketed to the consumer without the addition of a further dye or colourant.

The dentifrice compositions of the present invention may also be used with the addition of a further dye or colourant as desired.

A dentifrice composition of the present invention may be aqueous or non-aqueous. Water employed in the preparation of commercially suitable compositions should preferably be of low ion content and free of organic impurities. When present water will generally comprise from about 5 % to about 70 % such as from 10 % to 50 % by weight of the composition. Generally the level of water is up to 40 %, such as up to 36 %, such as from 0.1 % to 35 % by weight of the composition. The amount of water includes free water which is added plus that which is introduced with other materials, such as with humectants or surfactants.

A dentifrice composition of the present invention may further comprise a water-soluble condensed phosphate salt, such as an alkali metal pyrophosphate, tripolyphosphate or higher polyphosphate salt, in particular a water-soluble alkali metal tripolyphosphate salt.

Suitably the sodium form of this salt is preferred, although the potassium or mixed sodium and potassium salts could be used as a preferred embodiment as well. All physical forms can be used, e.g. a hydrate or a dehydrated form.

Most suitably the water-soluble alkali metal tripolyphosphate salt is sodium tripolyphosphate.

Suitably the water soluble condensed phosphate salt (such as an alkali metal tripolyphosphate salt) is present in an amount from 1.0 % to 20.0 %, for example from 2.0 % to 15.0 %, or 1.0 % to 10 %, or 5.0 % to 10.0 % by weight of the composition.

A dentifrice composition of the present invention may further comprise an alkali metal bicarbonate salt. The inclusion of such a salt in a dentifrice composition is beneficial for several reasons such as for providing good plaque removing capabilities, as well as for improving the whitening properties of dentifrices. Importantly bicarbonate salts provide a clean fresh feeling in the oral cavity after brushing and rinsing with water. Suitably the alkali bicarbonate is sodium bicarbonate. Suitably the sodium bicarbonate is present in an amount from 20 % to 90 % by weight of the composition, preferably from 60 % to 80 % by weight of the composition, more preferably from 65 % to 70 % by weight of the composition e.g. from 66 % to 68 % by weight of the composition.

A dentifrice composition of the present invention may comprise one or more active agents conventionally used in dentifrice compositions, for example, a fluoride source, a desensitising agent, an anti-bacterial agent, an anti-plaque agent, an anti-calculus agent, an oral malodour agent, an anti-inflammatory agent, an anti-oxidant, an anti-fungal agent, a wound healing agent or a mixture of at least two thereof. Such agents may be included at levels to provide the desired therapeutic effect.

Examples of desensitising agents include a tubule blocking agent or a nerve desensitising agent and mixtures thereof, for example as described in WO02/15809 (Block). Examples of desensitising agents include a strontium salt such as strontium chloride, strontium acetate or strontium nitrate or a potassium salt such as potassium citrate, potassium chloride, potassium bicarbonate, potassium gluconate and especially potassium nitrate.

A desensitising agent such as a potassium salt is generally present in an amount ranging from 2 % to 8 % by weight of the composition, for example 5 % by weight of the composition.

In another embodiment the desensitising agent comprises an arginine calcium carbonate salt. Suitably the arginine salt is present in an amount ranging from 0.5 % to 30 % by weight of the composition, such as from 1 % to 10 % by weight of the composition or from 1 % to 10 % by weight of the composition such as from 2 % to 8 % by weight of the composition.

In one embodiment the desensitising agent comprises a bioactive glass. Suitably the bioactive glass consists of 45 % by weight silicon dioxide, 24.5 % by weight sodium oxide, 6 % by weight phosphorus oxide, and 24.5 % by weight calcium oxide. One such bioactive glass is available commercially under the trade name, NOVAMIN, also known as 45S5 BIOGLASS.

Suitably the bioactive glass is present in an amount ranging from 1% to 20% by weight of the composition, such as from 1 % to 15 % by weight of the composition, or from 1 % to 10 % by weight of the composition, or from 2 % to 8 % by weight of the composition.

In one embodiment the desensitising agent comprises a stannous salt such as stannous chloride or stannous fluoride. Stannous salts, through hydrolysis and oxidation reactions, form insoluble metal salts that precipitate in dentinal tubules and on the dentine surface to provide effective relief from dentine hypersensitivity. Stannous salts also provide a benefit against dental erosion, dental caries and plaque/gingivitis.

In a further embodiment the desensitising agent includes silica, colloidal silica, nano zinc oxide, sub-micron alumina and sub-micron polymer beads, in a fine particulate form comprising an average particle size in the range from 1nm to 5 µm.

Suitable sources of fluoride ions for use in the compositions of the present invention include an alkali metal fluoride such as sodium fluoride, an alkali metal monofluorophosphate such a sodium monofluorophosphate, stannous fluoride, or an amine fluoride in an amount to provide from 25 ppm to 3500 ppm of fluoride ions, preferably from 100 ppm to 1500 ppm. A typical fluoride source is sodium fluoride, for example the composition may contain 0.1 % to 0.5 % by weight of sodium fluoride, e.g. 0.204 % by weight (equating to 923 ppm of fluoride ions), 0.2542 % by weight (equating to 1150 ppm of fluoride ions) or 0.315 % by weight (equating to 1426 ppm of fluoride ions).

Such fluoride ions help promote the remineralisation of teeth and can increase the acid resistance of dental hard tissues for combating caries, dental erosion (i.e. acid wear) and/or tooth wear.

Compositions of the present invention will contain additional formulating agents such as, surfactants, humectants, non-abrasive (thickening) silicas, flavouring agents, sweetening agents, opacifying or colouring agents, preservatives and water, selected from those conventionally used in the oral hygiene composition art for such purposes.

Suitable surfactants for use in the present invention include anionic surfactants such as a sodium C₁₀₋₁₈alkyl sulphate, e.g. sodium lauryl sulphate. Sodium lauryl sulphate is generally considered to be anionic and strongly charged and is useful if high levels of foaming are desired when brushing teeth.

In addition to anionic surfactants, zwitterionic, amphoteric, cationic and non- or low-ionic surfactants may be used to aid foaming characteristics. When anionic and amphoteric surfactants are used together an optimised foaming system is achieved that will provide both improved mouth feel and good cleaning. Examples of amphoteric surfactants include long chain alkyl (e.g. C₁₀-C₁₈ alkyl) betaines, such as the product marketed under the trade name 'Empigen BB' by Albright & Wilson and long chain alkyl amidoalkyl betaines such as cocamidopropylbetaine.

A particularly preferred example of an anionic/amphoteric surfactant combination for use in the present invention is sodium lauryl sulphate/cocamidopropylbetaine.

Suitably, the surfactant is present in the range from 0.1 % to 15 % by weight of the composition, for example from 0.5 % to 10 % by weight of the composition or from 1.0 % to 5 % by weight of the composition.

Suitable humectants for use in compositions of the invention include glycerin, xylitol, sorbitol, propylene glycol or polyethylene glycol, or mixtures of at least two thereof; which humectant may be present in an amount ranging from 10 % to 80 % by weight of the composition, for example from 20 % to 70 % by weight of the composition, or from 30 % to 60 % by weight of the composition.

The dentifrice compositions of the present invention are typically formulated in the form of toothpastes, instant powders, tablets and gels. Preferred compositions of the present invention are toothpastes and gels.

The dentifrices of the present invention are typically formulated in the form of a paste that is suitable for containing in and dispensing from a laminate tube or a pump as conventionally used in the art. Additional examples may include bag-in-can or bag-on-valve delivery systems that utilise a foaming agent such as pentane or iso-pentane.

The invention is further illustrated by the following examples.

### Aqueous dentifrice example.

The following toothpaste was prepared by mixing together all of the ingredients.

| **Ingredient** | **Weight %** |
|---|---|
| Water | 35 |
| Sorbitol | 27 |
| Silica (abrasive) | 13 |
| Glycerol | 10 |
| Silica (thickening) | 9 |
| SLS | 2 |
| Thickening gum | 1 |
| Flavouring | 2.3 |
| Sodium Fluoride | 0.3 |
| **Fine alumina (D90 <5µm)** | 0.4 |

The paste had a pale white colour with the addition of 0.4% by weight of the fine alumina.

### Non-aqueous dentifrice example

The following non-aqueous paste was prepared.

| **Ingredient** | **Weight %** |
|---|---|
| Glycerol | 54.5 |
| PEG 400 | 20 |
| Dental silica | 8.40 |
| Hydrated silica | 6.0 |
| STP (sodium tripolyphosphate) | 5.0 |
| SLS | 1.10 |
| Betaine | 0.40 |
| Flavouring/sweetener | 2.58 |
| Stannous Fluoride | 0.45 |
| Sodium Fluoride | 0.07 |
| **Fine alumina (D90 <5µm)** | 1.50 |

With the addition of 1.50 % by weight of the fine alumina the paste acquires a brilliant white appearance.

## Claims

1. A dentifrice composition comprising fine ground alumina with a D90 particle size of less than 5 µm and an orally acceptable carrier.

2. The dentifrice composition according to claim 1 wherein the D90 particle size of the the fine ground alumina is less than 3 µm.

3. The dentifrice composition according to either claim 1 or claim 2 wherein the fine ground alumina has a bulk density of between 800 g and 1200 g per 1000 cm³.

4. The dentifrice composition according to either wherein the fine ground alumina comprises between 0.05 % and 15 % by weight.

5. The dentifrice composition of any of the previous claims wherein the fine ground alumina comprises between 0.1 % and 2 % by weight.

6. The dentifrice composition as claimed in any one of the previous claims wherein the fine ground alumina comprises between 0.2 % and 0.8 % by weight.

7. The dentifrice composition as claimed in any one of the previous claims wherein the dentifrice composition is either aqueous or non-aqueous.

8. The dentifrice composition as claimed in any one of the previous claim wherein the dentifrice composition further comprises an abrasive.

9. The dentifrice composition as claimed in any one of the previous claims wherein the dentifrice composition further comprises a water soluble condensed phosphate.

10. The dentifrice composition as claimed in any one of the previous claims wherein the dentifrice further comprises at least one surfactant.

11. The dentifrice composition as claimed in any of the previous claims wherein the dentifrice composition further comprises two or more surfactants.

12. The dentifrice composition of either claim 10 or claim 11 wherein the surfactant further comprises between 0.1 % and 5 % by weight of surfactant.

13. The dentifrice composition as claimed in any one of the previous claims wherein the composition further comprises a desensitizing agent, an anti-bacterial agent, an anti-plaque agent, an anti-calculus agent, an oral malodour agent, an anti-inflammatory agent, an anti-oxidant, an anti-fungal agent, a wound healing agent or a mixture of at least two thereof.

14. The dentifrice composition as claimed in any one of the previous claims further comprising a source of fluoride ions.

15. The dentifrice composition as claimed in any one of the previous claims wherein the composition is free of titanium dioxide.
